(19) Europäisches Patentamt
European Patent Office
Office européen des brevets

(11) Veröffentlichungsnummer: **0 290 987 B1**

# (12) EUROPÄISCHE PATENTSCHRIFT

(45) Veröffentlichungstag der Patentschrift: **23.11.94**

(51) Int. Cl.5: **C12N 15/01**, C12N 5/00, A01H 1/02, A01G 7/00, A01N 57/20

(21) Anmeldenummer: **88107373.8**

(22) Anmeldetag: **07.05.88**

## (54) Herbizidresistente Kulturpflanzen, Verfahren zu deren Selektion und deren Regeneration.

(30) Priorität: **13.05.87 DE 3715958**

(43) Veröffentlichungstag der Anmeldung:
**17.11.88 Patentblatt 88/46**

(45) Bekanntmachung des Hinweises auf die Patenterteilung:
**23.11.94 Patentblatt 94/47**

(84) Benannte Vertragsstaaten:
**AT BE CH DE ES FR GB GR IT LI LU NL SE**

(56) Entgegenhaltungen:
**EP-A- 0 154 204**
**EP-A- 0 242 246**
**EP-A- 0 270 353**
**WO-A-86/02097**
**US-A- 4 443 971**

**SCIENCE, Band 223, 16. März 1984, Seite 1148; R.S. CHALEFF et al.: "Herbicide-resistant mutants from tobacco cell cultures"**

(73) Patentinhaber: **Hoechst Schering AgrEvo GmbH**
**Gerichtstrasse 27**
**D-13342 Berlin (DE)**

(72) Erfinder: **Donn, Günter, Dr.**
**Sachsenring 35**
**D-6238 Hofheim am Taunus (DE)**

EP 0 290 987 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist möglich, Maispflanzen aus Kalluskulturen, die aus unreifen Embryonen gewonnen wurden, zu regenerieren [Green C.E., Philips, R. L., Crop Sci. 15, 417 (1975); EP 160 390; EP 177 738]. Dies gelang jedoch zunächst nur bei wenigen Genotypen, beispielsweise bei Inzuchtlinien A188, W64A, Black mexican sweet. Erst verbesserte Nährmedien erlaubten es, von einer Vielzahl unterschiedlicher Inzuchtlinien morphogene und embryogene Kalluskulturen zu etablieren [Duncan et al., Planta 165, 322 (1985)]. Wichtige Voraussetzung hierfür ist der Zusatz von Aminosäuren zum Nährmedium.

Zellkulturen, die sich über einige Monate oder Jahre zu intakten Pflanzen regenerieren lassen, eignen sich zur Selektion von Mutanten- und Varianten-Zellinien. Wird beispielsweise dem Nährmedium eine toxische Dosis eines Herbizids zugesetzt, überleben einige wenige Kallus-Sektoren, aus denen herbizidresistente Pflanzen regeneriert werden können. Wird das Kallusgewebe mit Mutagenen behandelt, erhöht sich die Mutantenausbeute (US 4,443,971).

So wird in der WO-A-8603097 ein Verfahren zur Selektion einer gegen Phosphinothricin resistenten Alfalfa-Zellinie beschrieben.

Herbizide, die Inhibitoren von Enzymen sind, die wiederum an der Biosynthese von Aminosäuren beteiligt sind, entfalten ihre herbizide Wirkung auf Zellkulturen nur in abgeschwächter Form, wenn das Medium Aminosäuren enthält, insbesondere wenn es sich um Aminosäuren handelt, deren Biosynthese durch das entsprechende Herbizid inhibiert wird. Daher ist es in Gegenwart von Aminosäuren, unter Bedingungen also, die man bislang für die Etablierung und Subkultivierung eines regenerationsfähigen Maiskallus als besonders günstig erachtete, sehr schwierig, gleichzeitig auch Mutanten zu finden, die gegenüber Inhibitoren, welche die Aminosäurebiosynthese hemmen, resistent sind.

Überraschend wurde gefunden, daß regenerationsfähige Zellinien von Kulturpflanzen in Nährmedien kultiviert werden können, die keine Aminosäuren enthalten. Auch die Regeneration von Kulturpflanzen aus diesen Zellinien ist in derartigen Nährmedien möglich. Dies war insbesondere unerwartet, da Aminosäuren für Wachstum und Regeneration der Kalluskulturen bzw. der Pflanzen wesentlich sind. Derartige Kulturen können zur Selektion von herbizidresistenten Zellinien Kalluskulturen bzw. Pflanzen eingesetzt werden.

Die Erfindung betrifft somit ein Verfahren zur sicheren Selektion von Mais-Zellinien, die gegen Inhibitoren resistent sind, welche die Glutaminsynthetase hemmen, durch Kultivierung auf inhibitorhaltigen Nährmedien, das dadurch gekennzeichnet ist, daß

a) Kalluskulturen oder Zellsuspensionskulturen selektioniert werden, die auf aminosäurefreien Nährmedien unter Beibehaltung ihrer embryogenen und morphogenen Kompetenz wachsen,

b) diese Kulturen auf aminosäurefreiem, inhibitorhaltigen Nährmedium kultiviert und inhibitorresistente Kulturen selektioniert werden, aus denen intakte Pflanzen regeneriert werden können.

Im folgenden wird die Erfindung detailliert beschrieben, insbesondere in ihren bevorzugten Ausführungsformen. Ferner wird die Erfindung in den Ansprüchen definiert.

Mit Hilfe des im folgenden beschriebenen erfindungsgemäßen Verfahrens können regenerationsfähige, herbizidresistente Zellinien von Maiszellinien hergestellt werden.

Um herbizidresistente Zellkulturen selektieren zu können, ist es notwendig, zuerst Zellinien zu etablieren, die auf aminosäurefreien Medien hinreichend gut vermehrt werden können und außerdem in großer Frequenz zur Regeneration der Pflanze induzierbar sind. Dazu werden Kalluskulturen auf aminosäurefreiem Medium, beispielsweise modifiziertes Murashige Skoog (MS) Medium [Physiol. Plant. 15, 473 (1962)] oder modifiziertes $N_6$-Medium [Chu C. C. et al., Sci. Sin. 16, 659 (1975)], kultiviert. Derartige Nährmedien enthalten im wesentlichen eine Kohlenstoffquelle, wie z.B. Saccharose, Glucose, Maltose und Raffinose, eine Stickstoffquelle, beispielsweise Ammoniumsalze oder Nitrate, sowie die dem Fachmann bekannten Vitamine, Hormone und Mineralsalze.

Bevorzugt wird den betreffenden Nährmedien ein oder mehrere physiologische organische Säuren bzw. deren Salze, die das Wachstum der Kulturen positiv beeinflussen, zugegeben, insbesondere Säuren des Citratcyklus, wie z.B. Citronensäure, Äpfelsäure, Oxalessigsäure, Bernsteinsäure, und Brenztraubensäure, oder deren Salze. Als Salze werden vorzugsweise Natrium-, Kalium- oder besonders bevorzugt Ammoniumsalze verwendet. Diese Zusätze begünstigen das Wachstum von Kallus- bzw. Zellsuspensionskulturen auf den aminosäurefreien Medien und ermöglichen eine Subkultivierung unter Standardkulturbedingungen über einen langen Zeitraum, mindestens 1 bis 2 Jahre, ohne Verlust der Pflanzenregenerationsfähigkeit. Diese Verbindungen können daher dem Nährmedium als Ersatz für Aminosäuren zugesetzt werden, vorzugsweise in Konzentrationen von etwa 0,1 bis 10 mmol, insbesondere 0,5 bis 2 mmol. Unter Standardkulturbedingungen sind für den Fachmann übliche Bedingungen zu verstehen. Es kann bei ca. 15 bis 35 °C, bevorzugt 20 bis 30 °C, kultiviert werden, sowohl mit oder ohne Beleuchtung. Als Transferintervalle werden üblicherweise ca. 10 bis 30 Tage gewählt, bevorzugt 14 bis 21 Tage, was natürlich von dem

Wachstum der Kulturen abhängig gemacht werden muß.

Zellinien, die auf aminosäurefreien Nährmedien wachsen können, werden dann zur Selektion inhibitorresistenter, insbesondere herbizidresistenter Kalluskulturen und Zellsuspensionskulturen verwendet. Mit diesen Kulturen kann gegen grundsätzlich alle Inhibitoren selektiert werden, bevorzugt wird jedoch gegen Herbizide selektiert, die die Glutaminsynthetase hemmen. Innerhalb dieser Gruppe wird wiederum bevorzugt gegen Herbizide mit der allgemeinen Formel I

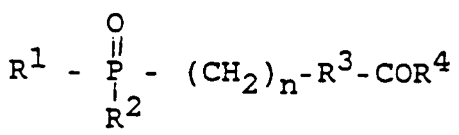

I

selektiert, in der unabhängig voneinander

- $R^1$ Hydroxyl oder Methyl,
- $R^2$ Hydroxyl, Methyl oder Ethyl ist und
- $R^3$ Aminomethylen, Hydroxymethylen oder eine Carbonylgruppe,
- $R^4$ OH oder

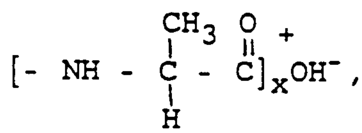

wobei x 1 bis 5, bevorzugt 1 bis 2, sein kann, und

n 0 bis 4, bevorzugt 0 bis 2, bedeutet. Insbesondere bevorzugt sind Glufosinate (Phosphinothricin) bzw. dessen Strukturanaloge, wie Bialaphos, sowie Dimethylphosphinylhydroxyessigsäure. Wenn es sich um optisch aktive Inhibitoren handelt, kann man sowohl das Racemat der Verbindungen als auch das biologisch aktive Enantiomer verwenden.

Um bessere Ausbeuten bei der Selektion zu erhalten, können die ausgewählten Zellinien nach an sich bekannten Methoden mutagenisiert werden. Dies kann sowohl mit Chemikalien geschehen, wie beispielsweise Ethylmethansulfonat oder N-Methyl-N-nitrosoguanidin (MNG), oder auch durch Bestrahlung, wie z.B. mit Röntgen- oder UV-Strahlen. Man verwendet derartige Mutagene in Konzentrationen, so daß 30 bis 70 % der Zellen abgetötet werden.

Die eigentliche Selektion der inhibitorresistenten Zellsuspensionskulturen, vorzugsweise Kalluskulturen, geschieht auf den schon erwähnten aminosäurefreien Nährmedien, denen der Inhibitor zugegeben wurde. Seine Konzentration im Medium kann in weiten Bereichen schwanken und wird im wesentlichen so gewählt, daß 70 bis 99 %, bevorzugt 95 bis 99 %, der Kalli absterben. Die Selektion kann mehrmals bei gleicher oder steigender Herbizidkonzentration wiederholt werden. Die Ausbeute der Selektion von resistenten Zellen bzw. regenerationsfähigen Kalli kann erhöht werden, wenn dem Nährmedium die oben aufgeführten, physiologischen organischen Säuren oder deren Salze zugegeben werden. Ferner kann die Selektionsschärfe durch Kultivierung der Kalluskulturen im Licht, ca. 8 bis 16 h bei 500 bis 5000 lux, verstärkt werden. Ansonsten wird die Kultivierung unter den üblichen Bedingungen über eine oder mehrere Passagen, bevorzugt 2 bis 4 Passagen, durchgeführt und ist sowohl auf Agar- als auch Flüssigmedien möglich. Transferintervalle schwanken je nach Geschwindigkeit des Wachstums und dauern üblicherweise 1 bis 5 Wochen, vorzugsweise 3 bis 4 Wochen.

Insbesondere durch wiederholte Subkultivierung der resistenten, morphogenen Kalli und Zellsuspensionen können Zellinien etabliert werden, die Herbizidkonzentrationen bis zu 5 mM tolerieren und noch zur Pflanzenregeneration befähigt sind.

Mit Hilfe des erfindungsgemäßen Verfahrens können natürlich auch Zellsuspensionskulturen und regenerationsfähige Kalluskulturen selektioniert werden, die gegen zwei Herbizide mit unterschiedlichem Wirkort resistent sind.

Die Regeneration der herbizidresistenten Pflanzen erfolgt nach an sich bekannten Methoden auf Nährmedien, die ebenfalls das Herbizid enthalten können, gegen das vorher selektioniert wurde. Unter Umständen kann jedoch eine Regeneration auf Nährböden, die kein Herbizid enthalten, vorteilhaft sein. Die Erfindung betrifft somit auch herbizidresistente Kulturpflanzen, die nach den vorstehend aufgeführten Kultivierungsmethoden erhältlich sind, sowie die Verwendung dieser Pflanzen zur Kreuzung mit anderen

Genotypen, wobei auch dieser Teil der Erfindung sich bevorzugt auch einkeimblättrige Kulturpflanzen, insbesondere Getreidepflanzen, und besonders bevorzugt auf Maispflanzen bezieht.

Mit Hilfe von Kreuzungen können beispielsweise auch Hybriden erzeugt werden, die gegenüber einem oder zwei Herbiziden resistent sind. Dies erreicht man durch Kreuzung einer herbizidresistenten Pflanze mit einer nicht resistenten Inzuchtlinie bzw. durch Kreuzung von zwei herbizidresistenten Pflanzen, die gegenüber unterschiedlichen Herbiziden mit unterscheidbarem Wirkort selektiert wurden. Derartige Kreuzungen können ebenfalls nach bekannten Methoden durchgeführt werden. Unter herbizidresistenten Pflanzen sind solche zu verstehen, die bei der doppelten Applikationsmenge, die zur Unkrautbekämfpung notwendig ist, keine Schadsymptome zeigen.

Die Erfindung betrifft ferner ein Verfahren zum Schutz der Kulturpflanzen, indem selektiv Unkraut mit einem Herbizid vernichtet wird, auf Anbauflächen, die mit Pflanzen, die nach dem erfindungsgemäßen Verfahren erhältlich und damit gegen das Herbizid resistent sind, bepflanzt werden. Als Herbizid werden bevorzugt Stoffe eingesetzt, welche die Aminosäurebiosynthese hemmen, insbesondere Verbindungen mit der oben aufgeführten allgemeinen Formel I. Besonders bevorzugt werden Glufosinate bzw. dessen Strukturanaloge, wie beispielsweise Di- und Tripeptide, sowie Dimethylphosphinylhydroxyessigsäure. Wenn es sich um optisch aktive Inhibitoren handelt, kann sowohl das Racemat als auch die eigentlich optisch aktive Verbindung verwendet werden. Das Herbizid bzw. Gemische der Herbizide werden in an sich bekannter Weise auf die Anbaufläche aufgebracht, möglicherweise in Intervallen, bevorzugt ungefähr 10 bis 100 Tage nach der Aussaat der Kulturpflanzen, bis das Unkraut genügend unterdrückt ist.

Im folgenden wird die Erfindung anhand von Beispielen näher erläutert.

## Beispiele

1. **Etablierung morphogener Maiszellkulturen auf aminosäurefreiem Nährmedium**

a) Aus unreifen Maiskörnern der Inzuchtlinien B73, W64A, A 188 (deren Hybriden konnten ebenfalls eingesetzt werden) wurden 10 bis 14 Tage nach der Bestäubung der weiblichen Blüten junge Embryonen herauspräpariert. Vorzugsweise wurden Embryonen, die 1 bis 1,5 mm lang waren, unter aseptischen Bedingungen auf Kallusinduktionsmedium transferiert und bei 25 °C ± 2 °C im Dunkeln kultiviert. Als Kallusinduktionsmedium eignen sich

A: ein modifiziertes Medium nach Murashige und Skoog oder

B: ein modifiziertes $N_6$-Medium,

deren Zusammensetzung in Tabelle 1 wiedergegeben ist, unter Zusatz von Prolin (1500 mg/l), Asparagin (500 mg/l), Glutamin (500 mg/l), Caseinhydrolysat (vitaminfrei, 500 mg/l) und Saccharose (60 g/l).

Die Nährmedien wurden mit 0,7 % Agar versetzt und vor dem Autoklavieren mit KOH auf einen pH-Wert von 5,8 eingestellt. Die Vitamine wurden sterilfiltriert und dem abgekühlten Medium zugegeben.

Tabelle 1: Nährmedien

| | modifiziertes MS-Medium (A) mg/l | modifiziertes $N_6$-Medium (B) mg/l |
|---|---|---|
| $MgSO_4 \cdot 7H_2O$ | 370 | 250 |
| $CaCl_2 \cdot 2H_2O$ | 440 | 166 |
| $KNO_3$ | 1900 | 2830 |
| $(NH_4)_2SO_4$ | - | 433 |
| $NH_4NO_3$ | 1650 | - |
| $KH_2PO_4$ | 170 | 400 |
| $MnSO_4$ | 22,3 | Die in der Klammer zusammengefaßten Verbindungen des MS-Mediums werden auch dem $N_6$-Medium zugesetzt. |
| KJ | 0,86 | |
| $CoCl_2 \cdot 6H_2O$ | 0,025 | |
| $ZnSO_4 \cdot 7H_2O$ | 8,6 | |
| $CuSO_4 \cdot 5H_2O$ | 0,025 | |
| $H_3BO_3$ | 6,2 | |
| $Na_2MoO_4 \cdot 2H_2O$ | 0,25 | |
| EDTA | 37,3 | |
| $FeSO_4 \cdot 7H_2O$ | 27,3 | |
| Thiamin·HCl | 0,5 | |
| Nicotinsäure | 0,2 | |
| Cyanocobalamin | 0,1 | |
| Pyridoxin HCl | 0,2 | |
| p-Aminobenzosäure | 0,05 | |
| Ca-Panthothenat | 0,1 | |
| Biotin | 0,1 | |
| Folsäure | 0,05 | |
| Cholin HCl | 0,1 | |
| Riboflavin | 0,05 | |
| Inosit | 100 | |
| Saccharose | 30000 | |
| 2,4-Dichlorphenoxyessigsäure | 1 mg | |
| oder 3,6-Dichloroorthoanissäure | 2 mg | |

Innerhalb von 2 bis 3 Wochen bildeten sich Kalluskulturen, die zur Bildung von Sproßprimordien und somatischen Embryonen befähigt waren.

Anschließend wurden diese Kalluskulturen auf aminosäurefreien Nährmedien A und B kultiviert. Ausgehend von 1500 Embryonen wurden 5 Zellinien etabliert, die auf derartigen Nährböden über mehr als ein Jahr unter Beibehaltung der Regenerationsfähigkeit wachsen konnten. Subkultivierung erfolgte im Abstand von 15 bis 28 Tagen.

b) Man verfährt gemäß Beispiel 1 a). Es wurden den aminosäurefreien Nährmedien jedoch folgende organische Säuren zugesetzt: Citronensäure (200 mg/l), $\alpha$-Ketoglutarat (150 mg/l), Äpfelsäure (130

mg/l), Oxalacetat (130 mg/l), Bernsteinsäure (120 mg/l) und Brenztraubensäure (90 mg/l). Die Stammlösung der organischen Säuren wurde vor der Zugabe zum Medium mit $NH_3$-Lösung auf pH 5,8 eingestellt.

2. **In vitro Mutagenese**

Morphogene Maiskallusstücke, die auf aminosäurefreiem Nährmedium wachsen können, wurden in flüssigem Salzmedium, bestehend aus den Salzen der Nährlösung B, mit 0,1 bis 1 % Ethylmethansulfonat 10 bis 120 min. inkubiert, 3 x mit dem Salzmedium in Abständen von 10 min. gewaschen und dann auf den Kulturmedien A oder B kultiviert. Die überlebenden morphogenen Kallussegmente wurden nach 2 bis 4 Wochen auf frischem Medium subkultiviert. Nach weiteren 2 bis 4 Wochen konnten die Kalli für Selektionsexperimente herangezogen werden.

3. **Selektion herbizidresistenter Kalluskulturen**

Es wurde ermittelt bei welcher Herbizidkonzentration im aminosäurefreien Kulturmedium A oder B (gemäß Beispiel 1b) 95 bis 99 % der Kalli starben. Sowohl für Glufosinate als auch für Dimethylphosphinyl-hydroxyessigsäure war dies bei einer Konzentration von $2 \times 10^{-4}$ mol/l der Fall.

Es wurden jeweils 10000 Kalli auf die herbizidhaltigen Agar-Medien (0,8 % Agar} transferiert und nach 6 bis 8 Wochen evaluiert. Die Petrischalen wurden bei 12 h Beleuchtung pro Tag mit 1000 bis 2000 Lux und 25 °C inkubiert. Auf Medien mit Glufosinate bildeten sich 12 Kalli aus, die unter Ausbildung dunkelgrüner Sproßprimordien wachsen. Auf Medien mit Dimethylphosphinyl-hydroxyessigsäure wurden 3 solcher Kalli erhalten.

Durch wiederholte Subkultur der resistenten morphogenen Kalli konnten Zellinien etabliert werden, die Herbizidkonzentrationen bis zu 5 mmol/l tolerieren und noch zur Pflanzenregeneration befähigt waren.

4. **Pflanzenregeneration**

Auf Regenerationsmedium (Medium A oder B ohne 2,4-Dichlorphenoxyessigsäure oder Dicamba) differenzierten die erhaltenen embryogenen herbizidresistenten Kalli innerhalb von 3 bis 5 Wochen, meist innerhalb von 3 bis 4 Wochen, vollständige Pflanzen aus. Sobald die Blätter eine Länge von 1 bis 3 cm erreichten, wurden die Pflanzen aus dem Agar in ein mineralisches Kultursubstrat (Vermiculit, Perlit) transplantiert und während der ersten 4 bis 7 Tage bei 90 bis 100 % relativer Feuchte kultiviert. Danach konnten die Pflanzen entweder in der Klimakammer oder im Gewächshaus weiterkultiviert werden. Bis zur Ausbildung von weiteren, 2 bis 4 Blättern, werden die Mais-Regenerate in Hydrokultur gehalten. Danach können die Pflanzen in Erde (sandiger Lehm) verpflanzt werden.

5. **Herbizidapplikation**

Im 4- bis 5-Blattstadium wurden die Pflanzen mit Herbizidlösungen in praxisüblichen Aufwandmengen (bei Glufosinate und Dimethylphosphinyl-hydroxyessigsäure 50 bis 200 mg Aktivsubstanz/$m^2$ entsprechend 0,5 bis 2 kg ai/ha) besprüht. Die Herbizide wurden als 0,1 bis 1 %ige wäßrige Lösungen appliziert. Die behandelten Pflanzen wurden nach 14 bis 28 Tagen visuell bonitiert. Der Test auf Herbizidresistenz wurde unter Bedingungen durchgeführt, die bei kommerziellen Maishybriden (Kontrollpflanzen) zu einer schweren Schädigung (Schädigungsgrad > 90 %) geführt haben. In Tabelle 2 sind die Ergebnisse der Herbizidapplikation an Regeneratpflanzen zusammengefaßt.

**Tabelle 2:**

**Herbizide Wirkung von Dimethylphosphinyl-hydroxyessigsäure und DL Glufosinate auf Regeneratpflanzen aus herbizidresistentem Kallus. Boniturergebnisse 4 Wochen nach der Applikation.**

| Appliziertes Herbizid | Aufwandmenge (kg ai/ha) | Schädigung in % | |
|---|---|---|---|
| | Wenn 2 Mengen angegeben: Splitapplikation in 10-tägigem Abstand | Kontrollpflanzen | Regeneratpflanzen aus resistentem Kallus |
| Dimethyl-phosphinyl-hydroxyessigsäure | 0 + 0 | 0 % | 0 % |
| | 0,5 + 1,0 | 95 % | 15 % |
| | 0,75 + 0,75 | 90 % | 10 % |
| | 1,0 + 0 | 75 % | 5 % |
| D,L-Glufosinate | 0 | 0 % | 0 % |
| | 0,75 | 90 % | 0 % |
| | 1,0 | 98 % | 10 % |

6. **Vererbbarkeit der an Regeneratpflanzen beobachteten Herbizidresistenz**
Ein Teil der Glufosinate behandelten Regeneratpflanzen entwickelten sich zu fertilen Pflanzen. Diese wurden geselbstet und als Hybridelter (Pollenspender) in Kreuzungsexperimenten mit herbizidsensitven (Wildtyp) Genotypen, beispielsweise Karat (Sorte I) oder Edo (Sorte II) verwendet. 6 Wochen nach der Bestäubung wurden die reifen Samen geerntet.

Die Anzucht der $F_1$-Generation erfolgte in einer Klimakammer bei 25 °C Tagestemperatur und 14 h, Beleuchtung mit 30000 Lux sowie 20 °C Nachttemperatur unter 60 % relativer Luftfeuchtigkeit. Die Pflanzen wurden in Einheitserde kultiviert. 14 Tage nach der Aussaat wurden die Pflanzen im 3- bis 4-Blattstadium mit Glufosinate-Ammonium (®BASTA, Fa. Hoechst AG, kommerzielle Formulierung) besprüht. Die getesteten Aufwandmengen entsprechen 0,75 und 1,5 kg ai/ha.

Tabelle 3 gibt die Boniturergebnisse 4 Wochen nach der Herbizidbehandlung wieder.

**Tabelle 3: Boniturergebnisse der $F_1$-Nachkommen Glufosinate-resistenter Mais-Regeneratpflanzen**

| | | | |
|---|---|---|---|
| Herbizidaufwandmenge: | | 0,75 kg ai/ha | 1,5 kg ai/ha |
| Genotyp | | Schädigung (%) | |
| Sorte I | | 80 % | 95 % |
| Sorte II | | 90 % | 100 % |
| Wildtyp-Regeneratpflanzen | | 95 % | 100 % |
| $F_1$-Selbstungsnachkommen Glufosinate-toleranter Regeneratpflanzen | von je 10 getesteten Pflanzen | 3 Pflanzen 85 %<br>7 Pflanzen 20 % | 2 Pflanzen 95 %<br>6 Pflanzen 40 %<br>2 Pflanzen 20 % |
| Kreuzung Sorte I x resistente Regeneratpflanzen | | 6 Pflanzen 85 %<br>4 Pflanzen 20 % | 7 Pflanzen 95 %<br>3 Pflanzen 30 % |

## Patentansprüche

**1.** Verfahren zur Selektion von Mais-Zellinien, die gegen Inhibitoren resistent sind, welche die Glutaminsynthetase hemmen, durch Kultivierung auf einem inhibitorhaltigen Nährmedium, dadurch gekennzeichnet, daß

a) Kalluskulturen oder Zellsuspensionskulturen selektioniert werden, die auf aminosäurefreiem Nährmedium unter Beibehaltung ihrer embryogenen und morphogenen Kompetenz wachsen,

b) diese Kulturen auf aminosäurefreiem, inhibitorhaltigen Nährmedium kultiviert und inhibitorresistente Kulturen selektioniert werden, aus denen intakte Pflanzen regeneriert werden.

**2.** Verfahren nach Anspruch 1, dadurch gekennzeichnet, daß dem Nährmedium ein oder mehrere physiologische organische Säuren oder deren Salze zugesetzt werden.

**3.** Verfahren nach Anspruch 2, dadurch gekennzeichnet, daß dem Nährmedium Säuren oder Salze des Citratcyklus sowie Brenztraubensäure zugesetzt werden.

**4.** Verfahren nach Anspruch 2 oder 3, dadurch gekennzeichnet, daß die Säuren und Salze in Konzentrationen von 0,1 bis 10 mmol/l zugesetzt werden.

**5.** Verfahren nach Anspruch 4, dadurch gekennzeichnet, daß die Säuren und Salze in Konzentrationen von 0,5 bis 2 mmol/l zugesetzt werden.

6. Verfahren nach einem oder mehreren der Ansprüche 1 bis 5, dadurch gekennzeichnet, daß als Inhibitor ein Herbizid zugesetzt wird.

**Claims**

1. A process for the selection of maize cell lines which are resistant to glutamine synthetase inhibitors by cultivation on an inhibitor-containing nutrient medium, which process comprises
   a) selecting callus cultures or cell suspension cultures which grow on amino acid-free nutrient medium while maintaining their embryogenic and morphogenic competence,
   b) cultivating these cultures on amino acid-free, inhibitor-containing nutrient medium, and selecting inhibitor-resistant cultures from which intact plants are regenerated.

2. The process as claimed in claim 1, which comprises adding one or more physiological organic acids or salts thereof to the nutrient medium.

3. The process as claimed in claim 2, which comprises adding acids or salts of the citrate cycle and pyruvic acid to the nutrient medium.

4. The process as claimed in claim 2 or 3, which comprises adding the acids and salts in concentrations of 0.1 to 10 mmol/l.

5. The process as claimed in claim 4, which comprises adding the acids and salts in concentrations of 0.5 to 2 mmol/l.

6. The process as claimed in one or more of claims 1 to 5, wherein the inhibitor added is a herbicide.

**Revendications**

1. Procédé pour la sélection de lignées cellulaires de maïs qui sont résistantes vis-à-vis des inhibiteurs, qui inhibent la glutamine-synthétase, par culture sur un milieu nutritif contenant des inhibiteurs, caractérisé en ce que
   (a) on sélectionne des cultures de cals ou des cultures de suspensions cellulaires, qui poussent sur des milieux nutritifs exempts d'acides aminés, tout en conservant leurs compétences embryogènes et morphogènes,
   (b) on cultive ces cultures sur des milieux nutritifs contenant des inhibiteurs, exempts d'acides aminés, et on sélectionne les cultures résistantes aux inhibiteurs, à partir desquelles on peut régénérer des plantes intactes.

2. Procédé selon la revendication 1, caractérisé en ce qu'on ajoute au milieu nutritif un ou plusieurs acides organiques physiologiques ou leurs sels.

3. Procédé selon la revendication 2, caractérisé en ce qu'on ajoute au milieu nutritif des acides ou des sels du cycle de citrate ainsi que l'acide pyruvique.

4. Procédé selon la revendication 2 ou 3, caractérisé en ce qu'on ajoute les acides et les sels en concentrations de 0,1 à 10 mmoles/litre.

5. Procédé selon la revendication 4, caractérisé en ce qu'on ajoute les acides et les sels en concentrations de 0,5 à 2 mmoles/litre.

6. Procédé selon une ou plusieurs des revendications 1 à 5, caractérisé en ce qu'on ajoute en tant qu'inhibiteur, un herbicide.